(19) Europäisches Patentamt
European Patent Office
Office européen des brevets

(11) Publication number: 0 021 292
B1

(12) EUROPEAN PATENT SPECIFICATION

(45) Date of publication of patent specification: 09.03.83

(21) Application number: 80103325.9

(22) Date of filing: 13.06.80

(51) Int. Cl.³: C 07 D 471/04,
A 61 K 31/505
//(C07D471/04, 239/00,
221/00)

# (54) Alkoxybenzylpyridopyrimidines, methods for their preparation and pharmaceutical formulations thereof.

(30) Priority: 14.06.79 GB 7920703
14.06.79 GB 7920704
14.06.79 GB 7920705
14.06.79 GB 7920706

(43) Date of publication of application:
07.01.81 Bulletin 81/1

(45) Publication of the grant of the patent:
09.03.83 Bulletin 83/10

(84) Designated Contracting States:
BE CH DE FR GB LI NL SE

(56) References cited:
GB - A - 913 710
GB - A - 970 583
GB - A - 1 084 103
US - A - 3 322 765

(73) Proprietor: THE WELLCOME FOUNDATION LIMITED
183-193 Euston Road
London NW1 2BP (GB)

(72) Inventor: Grivsky, Eugene Michael
4407 East Wood Court
Fairfax Virginia 22032 (US)
Inventor: Sigel, Carl William
11116 Bremerton Court
Raleigh North Carolina 27612 (US)
Inventor: Duch, David Stanley
1422 Lake Pine Drive
Cary North Carolina 27511 (US)
Inventor: Nichol, Charles Adam
1508 Ward Street
Durham North Carolina 27707 (US)

(74) Representative: Berg, Wilhelm, Dr. et al,
Dr. Berg, Dipl.-Ing. Stapf, Dipl.-Ing. Schwabe, Dr. Dr. Sandmair Mauerkircherstrasse 45
D-8000 München 80 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

Alkoxybenzylpyridopyrimidines, methods for their preparation and pharmaceutical formulations thereof

The present invention relates to 2,4-diaminopyrido(2,3-d)pyrimidines, to pharmaceutical formulations comprising such compounds and to their use in medicine.

U.K. Patent No. 1 084 103 (and corresponding U.S. Patent No. 3 322 765) discloses 2,4-diaminopyrido(2,3-d)pyrimidines of the general formula (I)

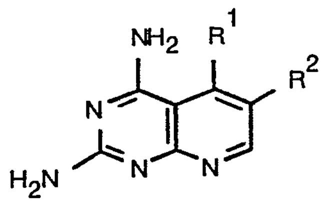

(I)

in which $R^1$ is an alkyl group and $R^2$ is an unsubstituted benzyl group or a benzyl group substituted by one or more halogen atoms, alkyl or alkoxy groups.

U.K. Patent No. 970 583 discloses compounds similar to those of formula (I) above but wherein $R^1$ is a hydrogen and $R^2$ is, *inter alia*, a phenalkyl group optionally bearing one or more substituents.

The compounds of formula (I) were described as having high *in vitro* and *in vivo* activity against bacteria or bacterial infections in experimental animals.

Subsequently it has been found that the compounds of formula (I) specifically disclosed in U.K. 1 084 103 show some inhibitory activity against mammalian dihydrofolate reductase (DHFR), and the activity was sufficient to render them potentially useful in the treatment of conditions where inhibition of mammalian DHFR is desirable.

It has further been found that many of these compounds are potent inhibitors of histamine N-methyltransferase (HMT), an enzyme involved in the metabolism of histamine. In this manner they often cause an undesirable accumulation of histamine in organs and tissues. The effects of histamine are well known and any possibility of a further utility for these compounds was substantially diminished by their strong inhibition of HMT.

Further investigation showed that a number of other compounds of formula (I) also posses DHFR inhibitory activity but that these, too, were also potent inhibitors of HMT. Others, which had acceptably low levels of inhibition of HMT were found to have insufficient activity as inhibitors of DHFR.

It has now been surprisingly found that certain 2,4-diamino-6-(dimethoxybenzyl)-5-methyl-pyrido(2,3-d)pyrimidines, which are within the scope of formula (I) but not specifically disclosed in U.K. 1 084 103, are not only very potent inhibitors of mammalian DHFR, but also have acceptably low inhibitory activity against HMT. These compounds are represented by formula (II) below and are useful in the treatment of proliferative diseases, such as psoriasis, basal and squamous cell carcinomas of the skin, and various forms of cancer including leukemias, lymphomas, sarcomas and solid tumors.

The invention accordingly provides compounds of formula (II):

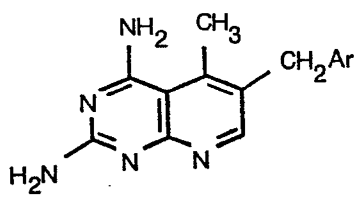

(II)

wherein Ar is 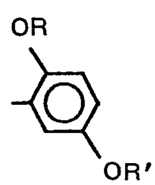 or 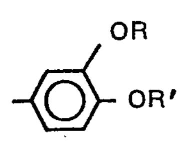

and R and R′ are lower ($C_1$—$C_6$) alkyl; and pharmaceutically acceptable acid addition salts thereof. Preferably monobasic salts are provided.

Preferably R and R′ are methyl. Particularly preferred is 2,4-diamino-5-methyl-6-(2,5-dimethoxy-benzyl)pyrido(2,3-d)pyrimidine.

The antiproliferative activity of the compounds of formula (II) resides in the free base and thus the nature of the acid participating in the acid addition salts is of minor importance. Such acid addition salts include, for example, those derived from hydrochloric acid, hydroiodic acid, sulphuric acid, phosphoric acid, acetic acid, p-toluenesulphonic acid, methanesulphonic acid, maleic acid, lactic acid, citric

acid, tartaric acid, succinic acid, oxalic acid, p-chlorobenzenesulphonic acid, isethionic acid, glucuronic acid, pantothenic acid and lactobionic acid.

The compounds of formula (II) may be prepared by any method known in the art for the preparation of compounds of analogous structure.

In particular the compounds of formula (II) be prepared by the reductive cleavage of the corresponding 7-substituted compound of formula (III):

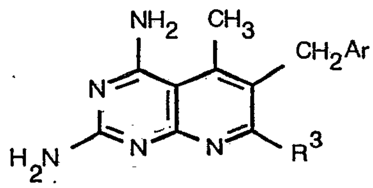

(III)

wherein Ar is as defined in formula (II) and $R^3$ is a leaving group capable of being removed by hydrogenolysis. Such groups include for example a mercapto or a halogeno (e.g. chloro) group.

Where, in a compound of formula (III), $R^3$ is SH the dethiation may for instance be conveniently effected by reaction with a desulphurizing agent such as Raney nickel or Raney cobalt or by catalytic hydrogenation utilizing hydrogen in the presence of a catalyst such as palladium on charcoal.

Compounds of formula (III) wherein $R^3$ is a mercapto group may be prepared from the corresponding 7-chloro compound (III) ($R^3 = Cl$) by reaction with a hydrosulfide as described in U.K. Patent No. 913 710 or by treatment of the corresponding 7-hydroxy compound with phosphorus pentasulfide.

In the case where $R^3$ is a halogen atom in the compounds of formula (III), the compounds of formula (II) may for instance be conveniently obtained by e.g. catalytic hydrogenation.

The compounds of formula (II) may also be prepared by reacting 2,4,6-triamino-pyrimidine (IV) with a compound of formula (V).

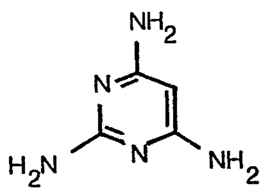

(IV) ; $CH_3\text{–A–}CH_2Ar$ (V)

wherein Ar is as defined in formula (II): A is 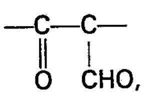 , 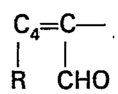 or 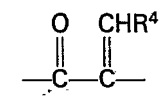

and $R^4$ is a leaving group such as for example a tertiary amino, alkoxy, alkylthio, halogeno, sulphonate or tosylate group.

The compounds of formula (II) may additionally be prepared by the conversion to amino groups, by methods known in themselves in pyrimidine chemistry, of the hydroxy and/or mercapto group(s) in a compound of formula (VI):

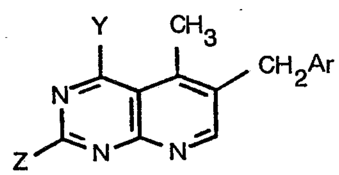

(VII)

in which Ar is as defined in formula (II), and Y and Z are the same or different and are OH, SH or $NH_2$ provided that at least one of Y and Z is OH or SH.

Compounds of formula (VI) maybe obtained by methods known in the art for preparing such compounds. In addition those in which Y is OH or $NH_2$ and Z is OH or SH may be obtained for example by reaction of urea, guanidine or thiourea with a suitable compound of formula (VII)

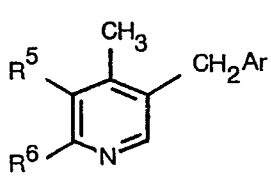

(VII)

in which Ar is as defined in formula (II);

$R^5$ is—$CO_2H$, $CO_2Alkyl$, —$CONH_2$ or CN and
$R^6$ is $NH_2$, Cl or Br.

While it is possible for the compounds of formula (II) or acid addition salts thereof (hereinafter referred to as the "active compounds") to be administered as the raw chemical they are preferably presented in the form of a pharmaceutical formulation.

The invention therefore further provides a pharmaceutical formulation comprising the active compound together with a pharmaceutically acceptable carrier therfor. The carrier must be "acceptable" in the sense of being compatible with the other ingredients of the formulation and not deleterious to the recipient thereof.

The pharmaceutical formulations may be prepared by a method comprising bringing into association an active compound and a pharmaceutically acceptable carrier therefor.

Topical application is particularly suitable when the active compounds are for use in the treatment of proliferative skin diseases.

The term "topical" as applied herein relates to the use of the active ingredient incorporated in a suitable pharmaceutical carrier, and applied at the site of the disease for the exertion of local action.

Pharmaceutical formulations suitable for topical administration may be presented in anhydrous forms such as ointments, lotions, pastes, jellies, sprays, aerosols, and bath oils. The term ointment includes formulations (including creams) having oleaginous, absorption, water soluble and emulsion type bases, for example petrolatum, lanolin, polyethylene glycols and mixtures thereof.

Topical formulations may contain a concentration of the active ingredient of from 0.05 to 2% w/w preferably 0.1 to 1% w/w most preferably 0.2 to 0.5% w/w.

Other pharmaceutical formulations include those suitable for oral, rectal and parenteral administration, although of these oral is preferred. The formulations may, where appropriate, be conveniently presented in discrete dosage units and may be prepared by any of the methods well known in the art of pharmacy. A convenient unit dose formulation contains the active compound in amount of from 50 mg to 1 g, preferably 100 mg to 500 mg, most preferably about 200 mg, to be taken once or several times daily.

All methods for the preparation of such formulations include the step of bringing into association the active compound with liquid carriers or finely divided solid carriers or both and then, if necessary, shaping the product into the desired formulation.

Pharmaceutical formulations suitable for oral administration wherein the carrier is a solid are most preferably presented as unit dose formulations such as boluses, capsules, cachets or tablets each containing a predetermined amount of the active compound. A tablet may be made by compression or moulding, optionally with one or more accessory ingredients. Compressed tablets may be prepared by compressing in a suitable machine the active compound in a free-flowing form such as a powder or granules optionally mixed with a binder, lubricant, inert diluent, lubricating, surface active or dispersing agent. Moulded tablets may be made by moulding an inert liquid diluent. Tablets may be optionally coated and, if uncoated, may be optionally scored. Capsules may be prepared by filling the active compound either alone or in admixture with one or more accessory ingredients, into the capsule cases and then sealing them in the usual manner. Cachets are analogous to capsules wherein the active ingredient together with any accessory ingredient(s) is sealed in a rice paper envelope.

Pharmaceutical formulations suitable for oral administration wherein the carrier is a liquid may be presented as a solution or a suspension in an aqueous liquid, or as an oil-in-water or water-in-oil liquid emulsion.

Pharmaceutical formulations suitable for rectal administration wherein the carrier is a solid are most preferably presented as unit dose suppositories.

Suitable carriers include cocoa butter and other material commonly used in the art, and the suppositories may be conveniently formed by admixture of the active compound with the softened or melted carrier(s) followed by chilling and shaping in moulds.

Pharmaceutical formulations suitable for parenteral administration include sterile solutions or suspensions of the active compound in aqueous or oleaginous vehicles. Such preparations are conveniently presented in unit dosage or multi-dose containers which are sealed after introduction of the formulation until required for use.

It should be understood that in addition to the aforementioned carrier ingredients the pharmaceutical formulations described above may include, as appropriate, one or more additional carrier ingredients such as diluents, buffers, flavouring agents, binders, surface active agents, thickeners, lubricants, preservatives (including anti-oxidants) and the like, and substances included for the purpose of rendering the formulation isotonic with the blood of the intended recipient.

As has been described above the compounds of the present invention are useful for the treatment of proliferative diseases. Thus these compounds may be employed in a method for the treatment of a proliferative disease in a mammal including man which comprises the administration of an effective, non-toxic amount of the active compound once or several times a day orally, or applied topically.

The invention also provides a compound of formula (II) as hereinbefore defined or an acid addition salt thereof for use in medicine.

The invention further provides a compound of formula (II) as hereinbefore defined or an acid addition salt thereof for use in the treatment of proliferative conditions.

The amount of active compound required for therapeutic effect as an antiproliferative agent will of course vary not only with the particular salt used but also with the route of administration. In general a suitable dose for the treatment of a mammal (including man) will lie in the range of from 0.1 to 100 mg per kilogram bodyweight (mg/kg) per day, preferably in the range of 2.0 to 50 mg/kg, more preferably in the range of 5 to 20 mg/kg.

Toxic manifestations attributable to the active compounds are typically those associated with folate depletion, such as bone marrow depression, megalobastic changes, and gastrointestinal ulceration. Calcium leucovorin (calcium salt of 5-formyl-5,6,7,8,-tetrahydrofolic acid) may be administered to effect reversal of these toxic manifestations or to prevent their occurrence. The administration of calcium leucovorin may be effected concurrently with treatment or at any stage thereof whenever toxic symptoms appear.

Thus, the haematological activity of the active compound can be prevented or reduced by the simultaneous administration of leucovorin. Consequently, tissue levels of the active compound may be safely raised by increasing the dose of the compound together with a simultaneous administration of leucovorin.

The following Examples, which illustrate the invention, and are not intended as a limitation thereof.

Example 1

*2,4-Diamino-5-methyl-6-(2,5-dimethoxybenzyl)pyrido-(2,3-d)-pyrimidine*

A mixture of 2,5-dimethoxybenzaldehyde (100 g), ethyl acetoacetate (84.5 g), anhydrous benzene (200 ml), piperidine (6 ml) and acetic acid (12 ml) was heated at reflux for 3 hours in an apparatus fitted with a Dean-Stark trap to collect the azeotropically distilled water. The reaction mixture was cooled, benzene (300 ml) added and the solution washed successively with water (100 ml), cold 0.1 N hydrochloric acid (200 ml), 5% aqueous sodium bicarbonate (200 ml) and dilute acetic acid (100 ml) and dried over anhydrous magnesiumsulphate. The solvent was evaporated under reduced pressure and the residual oil distilled, b.p. 169—170°C/0.3 mm Hg to give ethyl$\alpha$-(2,5-dimethoxybenzylidene)acetoacetate (104 g:64%) which solidified on standing (m.p. 68—69°C) was recrystallised from ethanol-pentane (mp. 72—73°C). A portion of the product (38 g) was reduced catalytically in the presence of 5% palladium on charcoal catalyst (Pd/C) in ethyl acetate (150 ml). The product, after removal of solvent, was purified by distillation under reduced pressure to give ethyl$\alpha$-(2,5-dimethoxybenzyl)acetoacetate, b.p. 146—148°C/0.3 mm Hg.

A mixture of ethyl$\alpha$-(2,5-dimethoxybenzyl)acetoacetate (21.2 g), 2,4,6-traminopyrimidine (10 g) and diphenyl ether (100 ml) was heated at 190—230°C for 1.5 hours in an apparatus fitted with a Dean-Stark trap and water-ethanol (4 ml) was collected. Methanol (200 ml) and ethanol (50 ml) were added to the cooled reaction mixture. The resulting solid was collected by filtration and treated with boiling water (1 l) to give 2,4-diamino-5-methyl-6(2,5-dimethoxybenzyl)-7-oxo-7,8-dihydropyrido-(2,3-d)pyrimidine (17 g) m.p. 325—326°C.

2,4-Diamino-5-methyl-6-(2,5-dimethoxybenzyl)-7-oxo-7,8-dihydropyrido(2,3-d) pyrimidine (8 g) was chlorinated by treatment with Vilsmeier reagent prepared by slowly adding thionyl chloride (28.6 g) in dry chloroform (25 ml) to a solution of dimethylformamide (17.5 g) in chloroform (100 ml) at 0—5°C. The cold mixture of the pyridopyrimidine and Vilsmeier reagent was stirred, gradually allowed to reach ambient temperature and then heated at reflux for 3 hours. It was then treated with ethanolic base (80 ml), maintaining the temperature at 25—30°C with cooling. The brown product formed was isolated, treated further with aqueous ammonia and then recrystallised from ethanol to give 2,4-diamino-5-methyl-6-(2,5-dimethoxybenzyl)-7-chloropyrido (2,3-d)pyrimidine m.p. 193—196°C (dec.).

The chloro compound (0.3 g) was dissolved in ethanol (200 ml) containing potassium hydroxide (0.2 g). 5% palladium on charcoal catalyst (0.2 g) was added and hydrogenation commenced. Reduction was complete after 48 hours and yielded 2,4-diamino-5-methyl-6-(2,5-dimethoxybenzyl)pyrido-(2,3-d) pyrimidine, m.p. 252—254°C.

Example 2

*2,4-Diamino-6-(3,4-dimethoxybenzyl)-5-methylpyrido(2,3-d) pyrimidine*

A mixture of 3,4-dimethoxybenzaldehyde (100 g), ethyl acetoacetate (78 g) and anhydrous benzene (100 ml) was heated at reflux for 3 hours, during which time the theoretical amount of water was removed by azeotropic distillation. The solvent was then removed under reduced pressure and the residue recrystallised from ethyl acetate/pentane to give ethyl$\alpha$-(3,4-dimethoxybenzylidene)acetoacetate (153 g) m.p. 72°C. (analytical sample from additional recrystallization (ethanol-pentane), m.p. 85—86°C.)

Ethyl$\alpha$(3,4-dimethoxybenzylidene)acetoacetate (50 g) was catalytically reduced in a Parr low pressure apparatus in ethyl acetate (150 ml) in the presence of 5% palladium on charcoal catalyst (Pd/C) (3 g). The catalyst was removed by filtration, the solvent was removed under reduced pressure

and the residual oil was distilled to give ethylα-(3,4-dimethoxybenzyl)acetoacetate (42 g; b.p. 162—164°C/0.4 mm Hg).

A mixture of ethylα-(3,4-dimethoxybenzyl)acetoacetate (42 g) and 2,3,6-triaminopyrimidine (21 g) in diphenyl ether (120 ml) was heated at 190—230°C for 2 hours during which time the ethanol and water formed were azeotropically removed. The reaction mixture was cooled and methanol (250 ml) and ethanol (50 ml) were added. The resulting solid was collected by filtration and treated with boiling water (1 l) to give 2,4-diamino-5-methyl-6-(3,4-dimethoxybenzyl)-7-oxo-7,8-dihydropyrido(2,3-d)pyrimidine (26 g), m.p. 346—348°C.

To a solution of dimethylformamide (17.5 g) in chloroform (75 ml) was added thionyl chloride (14 g) in chloroform (25 ml) at 0—5°C. The reaction mixture was allowed to heat to 55—60° for about 5 minutes and cooled to 0°C. To the reaction mixture was added over a 20 minute period at 0°C 2,4-diamino-5-methyl-6-(3,4-dimethoxybenzyl)-7-oxo-7,8-dihydropyrido(2,3-d) pyrimidine (8.2 g). Chloroform (50 ml) was added and the mixture was heated at reflux for 2.5 hours, cooled and ethanolic base (50 ml) was added. The resulting brown product was collected by filtration, treated with 2N sodium hydroxide and recrystallized from ethanol to give crude product (7.6 g). Additional recrystallisation from ethanol-water gave 2,4-diamino-5-methyl-6-(3,4-dimethoxybenzyl)-7-chloro-pyrido(2,3-d)pyrimidine (2 g) mp. 224—225°C (dec). This was added to ethanol (250 ml) containing potassium hyroxide (0.2 g). 5% palladium on charcoal catalyst (0.1 g) was added and hydrogenation commenced. Reduction was complete after 4 hours in a Parrlow pressure apparatus at 240 to 275 KPascals and gave 2,4-diamino-6-(3,4-dimethoxybenzyl)-5-methylpyrido(2,3-d)pyrimidine (0.1 g), m.p. 265—268°C (dec).

Example 3

*Inhibition of Mammalian Dihydrofolate Reductase DHFR by 2,4-Diamino-6-benzylpyrido(2,3-d)pyrimidines*

The inhibitory effect of the test compounds against DHFR partially purified from rat liver was determined. The results are given in Table 1 below. An $IC_{50}$ of $5 \times 10^{-8}$M or less is considered significant potency, and $IC_{50}$ of $1 \times 10^{-8}$M or less being particularly significant potency.

TABLE 1

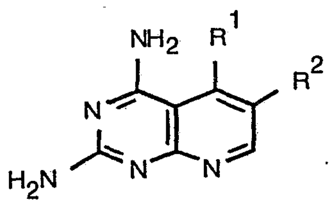

| TEST COMPOUND | | $IC_{50}$ $10^{-8}$ M |
|---|---|---|
| $R^1$ | $R^2$ | |
| $CH_3$ | $-CH_2Ph$ | 4 |
| $CH_3$ | 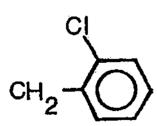 | 1.6 |
| $CH_3$ | 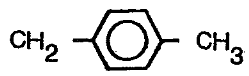 | 2.7 |
| $CH_3$ | 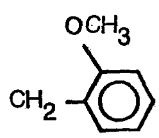 | 5.0 |
| $CH_3$ | 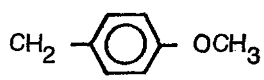 | 5.3 |
| $CH_3$ | 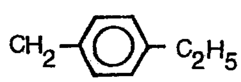 | 9 |
| H | 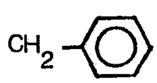 | 25 |
| $CH_3$ | 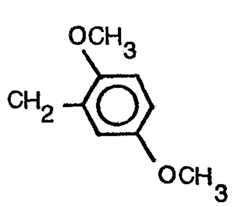 | 0.15 |
| $CH_3$ | 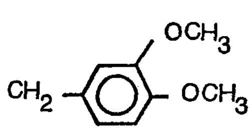 | 0.78 |

Example 4

*Inhibition of Histamine N-methyl-transferase (HMT) by 2,4-Diamino-6-benzylpyrido(2,3-d)pyrimidine*

The effect of the test compounds used in Example 3 against HMT was determined. The results are given in Table 2. An inhibition of less than 20% was considered acceptable and the lack of any effect on this enzyme is most desirable since any interference with histamine metabolism should be minimized.

TABLE 2

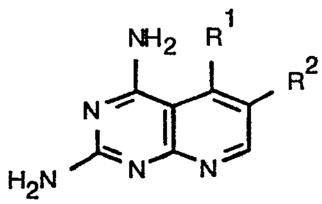

| TEST COMPOUND | | % inhibition of HMT at $10^{-5}$ M |
|---|---|---|
| $R^1$ | $R^2$ | |
| $CH_3$ | $-CH_2Ph$ | 51 |
| $CH_3$ | 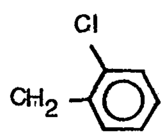 | 59 |
| $CH_3$ | 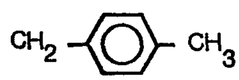 | Not Tested |
| $CH_3$ | 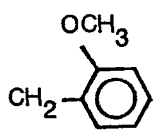 | 18 |
| $CH_3$ | 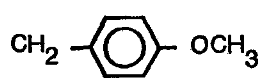 | 20 |
| $CH_3$ | 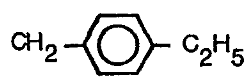 | 55 |
| H | 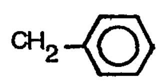 | 48 |
| $CH_3$ | 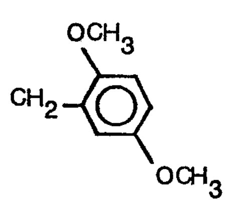 | 11 |
| $CH_3$ | 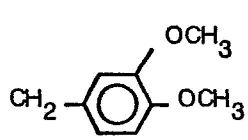 | 19 |

## Example 5

*Antitumor effect of 2,4-diamino-6-(2,5-dimethoxy-benzyl)-5-methylpyrido(2,3-d)pyrimidine*

Solid Walker 256 tumors were implanted subcutaneously into three groups of rats. On the third day after implantation with the tumors drug treatment was commenced and continued for 5 successive days. One group of rats were treated with 2,4-diamino-6-(2,5-dimethoxybenzyl)-5-methylpyrido(2,3-d)pyrimidine at a dose of 25 mg/kg q.i.d., a second group was treated with the same compound at 15 mg-kg b.i.d.; the third group were left untreated as controls. After 17 days the treated groups of rats showed mean tumor volumes of 29% and 10% respectively of the untreated group.

In a second study tumors were implanted subcutaneously and drug treatment delayed until 10 days after implantation. Drug treatment consisted of 30 mg/kg q.i.d. on days 10—13 and 50 mg/kg on days 20, 24 and 28 after tumor implantation. Mean tumor volumes were measured during the course of this study. Tumor volume in animals which received no treatment grew rapidly whereas those which received drug treatment showed a decrease in mean tumor volume during the period of observation. Twenty-three days after tumor implantation T/C was 0.03.

## Example 6

*Antileukemic effect of 2,4-diamino-6-(2,5-dimethoxybenzyl)-5-methylpyrido(2,3-d)pyrimidine*

Leukemia P388 cells were inoculated intraperitoneally in CDF, Charles River female mice, each animal receiving $10^6$ cells. On the second day after implantation with the leukemic cells, drug treatment was commenced and continued twice daily for three days. Four groups of mice were treated with 2,4-diamino-6-(2,5-dimethoxybenzyl)-5-methylpyrido(2,3-d)pyrimidine at doses of 22, 33.5, 50 and 75 mg/kg of body weight and a fifth group was left untreated as a control. All of the untreated mice died from the leukemia between the 10th and 12th day with a median survival of 11 days. All of the treated mice survived for periods longer than the untreated animals with a % increases in life span of 145%, 159%, 140% and 154% for the drug treated groups receiving 22, 33.5, 50 and 75 mg/kg doses respectively. This is a highly significant effect. There was no evidence of drug-related toxicity.

Within the same experiment, two closely related compounds were tested for antileukemic activity at the same dosage levels of 22, 33.5, 50 and 75 mg/kg, respectively, and with the same schedule of treatment. The corresponding pyridopyrimidine with a 4-methoxybenzyl substituent was inactive at all doses and the 2-chlorobenzyl compound had only marginal or slight activity. Thus, the 2,5-dimethoxybenzyl compound has substantially greater antileukemic activity than those compounds of similar structure.

## Example 7

*Water Soluble Ointment*

| | amount(g) |
|---|---|
| 2,4-Diamino-5-methyl-6-(dialkoxy-benzyl)pyrido(2,3-*d*)pyrimidine | 0.5 |
| Polyethylene glycol 300 | 20.0 |
| Polythylene glycol 1500 | 77.5 |
| Total | 100.0 |

## Example 8

*Skin Cream*

| | amount (g) |
|---|---|
| 2,4-Diamino-5-methyl-6-(dialkoxy-benzyl)pyrido(2,3-*d*)pyrimidine | 0.5 |
| Glyceryl monostearate | 20.0 |
| Methylparaben | 0.3 |
| Petrolatum light liquid | 4.0 |
| Propylene glycol | 5.0 |
| Span 60 | 2.0 |
| Tween 61 | 4.0 |
| Water | 64.2 |
| Total | 100.0 |

"Span" and "Tween" are Trade Marks.

Example 9

*Toxicity of 2,4-Diamino-5-methyl-6-(dimethoxybenzyl)pyrido(2,3-d)pyrimidines)*

The acute toxicity of the title compounds was determined in mice (i.p.). The following results were obtained:—

| 6-benzyl derivative | $LD_{50}$ (mg/kg bodyweight) |
|---|---|
| 2,5-dimethoxy | $\simeq$ 500 |

Example 10

*Tablet Formulation*

| | |
|---|---|
| 2,4-Diamino-5-methyl-6(dialkoxybenzyl) pyrido(2,3-d)pyrimidine | 100 mg |
| Starch | 50 mg |
| Lactose | 50 mg |
| Stearic acid | 3 mg |
| Gelatin | 15 mg |
| Total | 218 mg |

Example 11

*Injectable Formulation*

| | |
|---|---|
| 2,4-Diamino-5-methyl-6-(dialkoxybenzyl)-pyrido(2,3-d)pyrimidine q.s. to | 5 mg/ml |
| Propylene glycol | 40 ml |
| Ethanol | 11 ml |
| Water | 9 ml |

**Claims**

1. A compound of the formula (II):

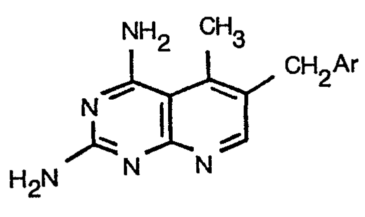

wherein Ar is 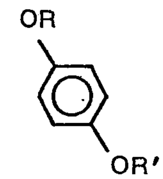 or 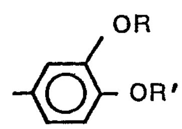

R and R′ are ($C_1$—$C_6$) alkyl, and acid addition salts thereof.

2. A compound of formula (II) as claimed in claim 1 wherein R and R′ are methyl.

3. 2,4-Diamino-6-(2,5-dimethoxybenzyl)-5-methylpyrido(2,3-d)pyrimidine and acid addition salts thereof.

4. A pharmaceutical formulation comprising a compound of formula (II) and acid addition salts thereof, together with a pharmaceutically acceptable carrier therefor.

5. A pharmaceutical formulation as claimed in claim 4 in unit dosage form.

6. A pharmaceutical formulation as claimed in claim 5 wherein the pyridopyrimidine is present in an amount of from 50 mg to 1 g.

7. A pharmaceutical formulation as claimed in claim 4 wherein the formulation is a topical formulation.

8. A pharmaceutical formulation as claimed in claim 7 wherein the pyridopyrimidine is present in an amount of 0.2% to 5% w/w.

9. A compound of formula (II):

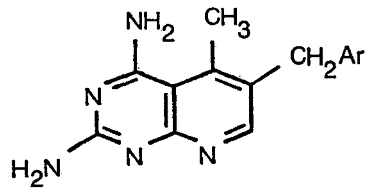

(II)

wherein Ar is 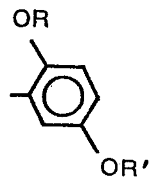 or 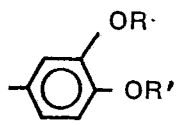

R and R′ are ($C_1$—$C_6$) alkyl and acid addition salts thereof for use as a therapeutic agent.

10. A compound as claimed in claim 9 for use in the treatment or prophylaxis of proliferative diseases.

11. A method for the preparation of a compound of formula (II):

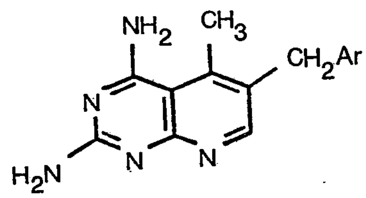

(II)

wherein Ar is 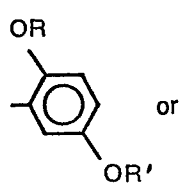 or 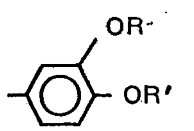

R and R′ are ($C_1$—$C_6$) alkyl and acid addition salts thereof, which method comprises:—

(A) reductive cleavage of a compound of formula (III)

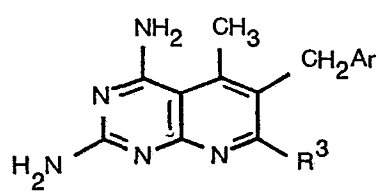

(III)

wherein Ar is as defined in formula (II) and $R^3$ is a leaving group capable of removal by hydrogenlysis;

(B) reacting 2,4,6-triaminopyrimidine with a compound of formula (V)

$$CH_3—A—CH_2—Ar \qquad (V)$$

wherein Ar is as defined in formula (II); A is 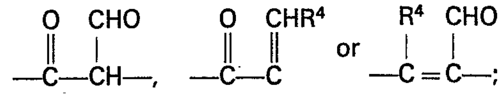 ; and $R^4$ is a leaving group; or

(C) Conversion to amino groups of the hydroxy and/or mercapto groups in a compound of formula (VI)

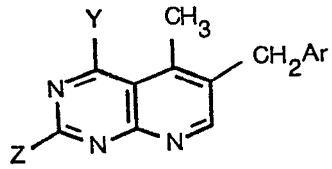

(VI)

wherein Y and Z are the same or different and are OH, SH or $NH_2$ provided that at least one of Y and Z is OH, or SH; and

Ar is as defined in formula (II);

**Revendications**

1. Composé de formule (II):

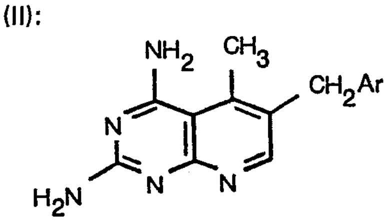

où Ar est 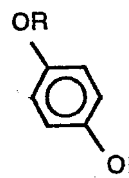 or 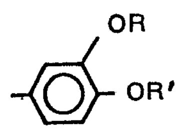

et R et R′ sont des alcoyles en ($C_1$—$C_6$); et ses sels d'addition d'acides.

2. Composé de formule (II) suivant la revendication 1, dans lequel R et R′ sont des méthyles.

3. La 2,4-diamino-6-(2,5-diméthoxybenzyl)-5-méthylpyrido(2,3-d)pyrimidine et sels d'addition d'acides.

4. Composition pharmaceutique comprenant un composé de formule (II) et ses sels d'addition d'acides, outre un véhicule pharmaceutiquement acceptable.

5. Composition pharmaceutique suivant la revendication 4, sous forme dosée unitaire.

6. Composition pharmaceutique suivant la revendication 5, dans laquelle la pyridopyrimidine est présente en quantité de 50 mg à 1 g.

7. Composition pharmaceutique suivant la revendication 4, qui est une composition à usage topique.

8. Composition pharmaceutique suivant la revendication 7, dans laquelle la pyridopyrimidine est présente en quantité de 0,2% à 5% p/p.

9. Composé de formule (II):

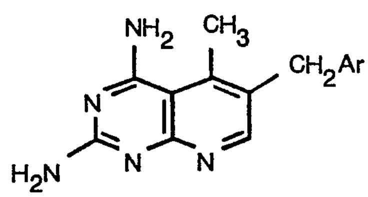

(II)

où Ar est 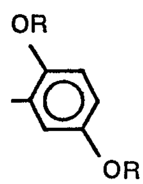 or 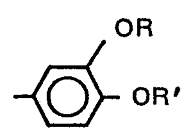

et R et R' sont des alcoyles en $(C_1—C_6)$; et ses sels d'addition d'acides, à utiliser comme agent thérapeutique.

10. Composé suivant la revendication 9, à utiliser pour le traitement ou la prophylaxie d'affections prolifératives.

11. Procédé de préparation d'un composé de formule (II):

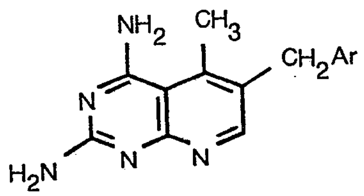

où Ar est 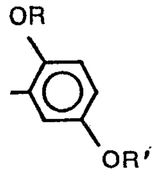 or 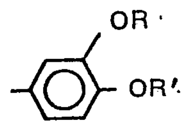

et R et R' sont des alcoyles en $(C_1—C_6)$; et de ses sels d'addition d'acides, qui comprend

(A) la scission réductrice d'un composé de formule (III)

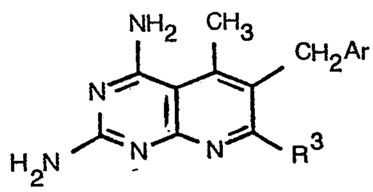

(III)

où Ar est tel que défini à propos de la formule (II) et $R^3$ est un radical labile susceptible d'élimination par hydrogénolyse;

(B) la réaction de la 2,4,6-triaminopyrimidine avec un composé de formule (V)

$$CH_3—A—CH_2—Ar \qquad (V)$$

où Ar est tel que défini à propos de la formule (II);

A est 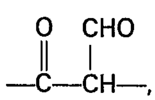, 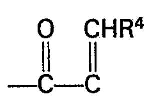 ou 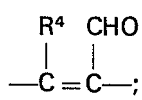; et $R^4$ est un radical labile ou

(C) la conversion en radicaux amino des radicaux hydroxyle et/ou mercapto d'un composé de formule (VI)

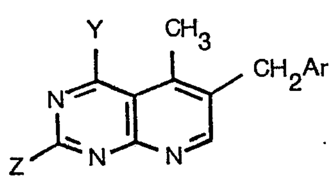

(VI)

où Y et Z sont identiques ou différents et sont OH, SH ou $NH_2$, étant entendu qu'au moins l'un d'entre Y et Z est OH ou SH, et

Ar est tel que défini à propos de la formule (II).

**Patentansprüche**

1. Verbindung der Formel (II)

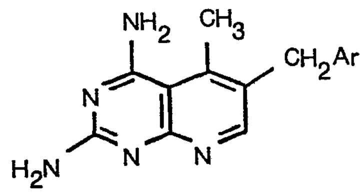

in der Ar für 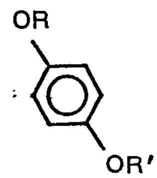 . 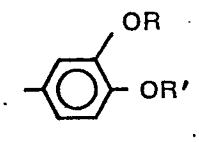

steht, worin R und R′ $(C_1—C_6)$-Alkylgruppen bedeuten, oder Säureadditionssalze davon.

2. Verbindung der Formel (II), wie sie in Anspruch 1 beansprucht ist, worin R und R′ Methylgruppen bedeuten.

3. 2,4-Diamino-6-(2,5-dimethoxybenzyl)-5-methylpyrido(2,3-d)pyrimidin und Säureadditionssalze davon.

4. Pharmazeutische Zubereitung enthaltend eine Verbindung der Formel (II) und Säureadditionssalze davon zusammen mit einem pharmazeutisch annehmbaren Träger dafür.

5. Pharmazeutische Zubereitung nach Anspruch 1 in Dosiseinheitsform.

6. Pharmazeutische Zubereitung nach Anspruch 5, worin das Pyridopyrimidin in einer Menge von 50 mg bis 1 g enthalten ist.

7. Pharmazeutische Zubereitung nach Anspruch 4, worin die Zubereitung eine topische Zubereitung ist.

8. Pharmazeutische Zubereitung nach Anspruch 7, worin das Pyridopyrimidin in einer Menge von 0,2% bis 5% (Gew./Gew.) vorhanden ist.

9. Verbindung der Formel (II)

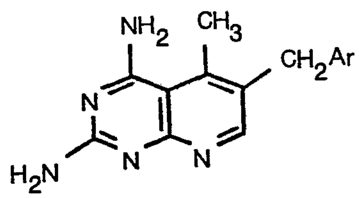

(II)

in der Ar für 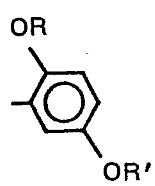 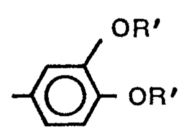

steht, worin R und R′ $(C_1—C_6)$-Alkylgruppen darstellen, und Säureadditionssalze davon zur Verwendung als therapeutischer Wirkstoff.

10. Verbindung nach Anspruch 9 zur Behandlung oder Prophylaxe von wuchernden Erkrankungen.

11. Verfahren zur Herstellung einer Verbindung der Formel (II)

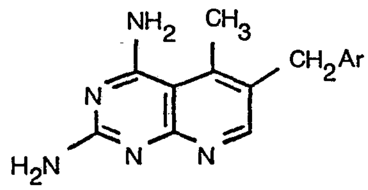

(II)

in der Ar für 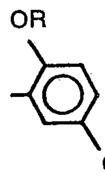 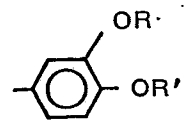

steht, worin R und R′ ($C_1$—$C_6$)-Alkylgruppen darstellen, oder Additionssalze davon, umfassend

(A) die reduktive Spaltung einer Verbindung der Formel (III)

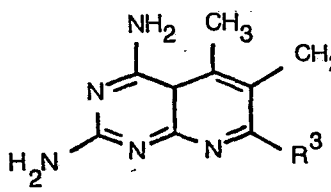

(III)

worin Ar die bezüglich der Formel (II) angegebenen Bedeutungen besitzt und $R^3$ eine austretende Gruppe darstellt, die durch Hydrogenolyse entfernt werden kann;

(B) die Umsetzung von 2,4,6-Triaminopyrimidin mit einer Verbindung der Formel (V)

$$CH_3—A—CH_2—Ar \qquad (V)$$

worin Ar die bezüglich der Formel (II) angegebenen Bedeutungen besitzt und A eine Gruppe der Formeln

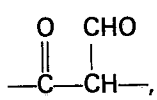, 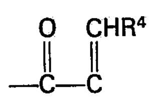 oder 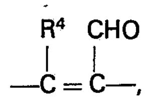,

worin $R^4$ für eine austretende Gruppe steht, darstellt; oder

(C) Umwandlung der Hydroxylgruppen und/oder Mercaptogruppen einer Verbindung der Formel (VI)

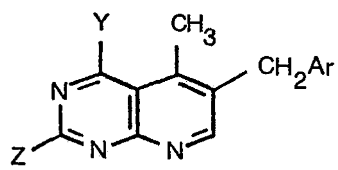

(VI)

worin Y und Z gleichartig oder verschieden sind und OH—, SH— oder $NH_2$-Gruppen darstellen, mit der Maßgabe, daß mindestens eine der Gruppen Y und Z eine OH— oder SH-Gruppe darstellt, und Ar die bezüglich der Formel (II) angegebenen Bedeutungen besitzt, in Aminogruppen.